(19) Europäisches Patentamt
European Patent Office
Office européen des brevets

(11) Veröffentlichungsnummer: **0 158 141 B1**

(12) # EUROPÄISCHE PATENTSCHRIFT

(45) Veröffentlichungstag der Patentschrift: **18.10.89**

(21) Anmeldenummer: **85102850.6**

(22) Anmeldetag: **13.03.85**

(51) Int. Cl.⁴: **A 61 K 6/00**, C 08 L 83/07 // (C08L83/07, 83:05)

---

(54) **Platin, Organopolysiloxan und Füllstoff enthaltende Pasten.**

---

(30) Priorität: **13.03.84 DE 3409139**

(43) Veröffentlichungstag der Anmeldung: **16.10.85 Patentblatt 85/42**

(45) Bekanntmachung des Hinweises auf die Patenterteilung: **18.10.89 Patentblatt 89/42**

(84) Benannte Vertragsstaaten: **CH DE FR GB LI**

(56) Entgegenhaltungen:
**AT-B- 209 484**
**AT-B- 355 199**
**DE-A- 2 508 560**
**DE-A- 2 736 421**
**DE-B- 2 249 822**

(73) Patentinhaber: **WACKER-CHEMIE GMBH, Prinzregentenstrasse 22, D-8000 München 22 (DE)**

(72) Erfinder: **Louis, Eckhart, Dr., Angererweg 20, D-8263 Burghausen (DE)**
Erfinder: **Hechtl, Wolfgang, Dr., Robert-Koch-Strasse 53, D-8263 Burghausen (DE)**
Erfinder: **Hefner, Heinz, Dr., Vivaldistrasse 2, D-88263 Burghausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Platin, Organopolysiloxan und Füllstoff enthaltende Pasten für die Bereitung von Zahnabdruckmassen aus Diorganopolysiloxan mit jeweils eine SiC-gebundene Vinylgruppe aufweisenden Triorganosiloxygruppen als endständigen Einheiten, mindestens 3 Si-gebundene Wasserstoffatome je Molekül enthaltendem Organopolysiloxan, Platin als Katalysator für die Anlagerung von Si-gebundenem Wasserstoff an die Vinylgruppen und Füllstoff als wesentlichen Bestandteilen sind bereits bekannt. Derartige Pasten wurden bisher den übrigen Bestandteilen der Massen im Gewichtsverhältnis von 7:1 bis 1:1 zugesetzt. Hierzu wird beispielsweise auf US 4 273 902, ausgegeben 16. Juni 1981, Kentanova Tomioka et al., G-C Dental Industrial Corp., verwiesen.

Es bestanden nun die Aufgaben, bei der Bereitung von Zahnabdruckmassen aus Diorganopolysiloxan mit jeweils eine Si-gebundene Vinylgruppen aufweisenden Triorganosiloxygruppen als endständigen Einheiten, mindestens 3 Si-gebundene Wasserstoffatome je Molekül enthaltendem Organopolysiloxan, Platin und Füllstoff als wesentlichen Bestandteilen insbesondere die Menge an schwer zugänglichem Platin zu senken, trotzdem aber bei Lagerung der Paste vor dem Vermischen der Paste mit den übrigen Bestandteilen der Masse eine geringere Änderung des Vernetzungsverhaltens der Masse als bisher zu erzielen, den Aufwand für die Prüfung grosser Mengen von Platin, Organopolysiloxan und Füllstoff enthaltenden Pasten zu vermeiden bzw. die Prüfung der weiteren Bestandteile der Masse zu vereinfachen, Platin, Organopolysiloxan und Füllstoff enthaltende Pasten bereitzustellen, die sich besonders rasch und leicht mit den übrigen Bestandteilen der Massen vermischen lassen und gleichzeitig Platin, Organopolysiloxan und Füllstoff enthaltende Pasten bereitzustellen, die auf den Händen keine oder praktisch keine Spuren hinterlassen, wenn die Pasten mit den übrigen Bestandteilen der Massen mit den blossen Händen unter Kneten vermischt werden.

Diese Aufgaben werden durch die Erfindung gelöst.

Gegenstand der Erfindung sind Platin, Organopolysiloxan und Füllstoff enthaltende Pasten für die Bereitung von Zahnabdruckmassen aus Diorganopolysiloxan mit jeweils eine SiC-gebundener Vinylgruppe aufweisenden Triorganosiloxygruppen als endständigen Einheiten, mindestens 3 Si-gebundene Wasserstoffatome je Molekül enthaltendem Organopolysiloxan, Platin und Füllstoff als wesentlichen Bestandteilen, dadurch gekennzeichnet, dass sie

a) Platin, berechnet als Element und bezogen auf das Gesamtgewicht der jeweiligen Platin enthaltenden und mit den übrigen Bestandteilen der Masse zu vermischenden Paste, in einer Menge von mindestens 100 Gewichts-ppm

b) 35 bis 75 Gewichtsprozent, bezogen auf das Gesamtgewicht der jeweiligen Platin enthaltenden und mit den übrigen Bestandteilen der Masse zu vermischenden Paste, Diorganopolysiloxan mit mindestens 2 SiC-gebundenen Vinylgruppen je Molekül und einer Viskosität von mindestens 100 mPa.s bei 25 °C,

c) 10 bis 25 Gewichtsprozent, bezogen auf das Gesamtgewicht der jeweiligen Platin enthaltenden und mit den übrigen Bestandteilen der Masse zu vermischenden Paste, von aliphatischen Kohlenstoff-Kohlenstoff-Mehrfachbindungen freie, bei Raumtemperatur flüssige oder verstreichbare Kohlenwasserstoffe und

d) 3 bis 15 Gewichtsprozent, bezogen auf das Gesamtgewicht der jeweiligen Platin enthaltenden und mit den übrigen Bestandteilen der Masse zu vermischenden Paste, hydrophobes Siliciumdioxyd mit einer Oberfläche von mindestens 50 $m^2/g$ enthalten, wobei die Summe der jeweils innerhalb der oben unter a) bis d) angegebenen Bereiche gewählten Prozentsätze einschliesslich der Prozentsätze von gegebenenfalls zusätzlich in der Paste vorliegenden Stoffen 100 Gewichtsprozent beträgt.

Das Platin (a) kann in beliebiger Form vorliegen, in der es in der bei Zahnabdruckmassen erforderlichen befriedigend kurzen Zeit im Munde des Patienten die Ablagerung von Si-gebundenem Wasserstoff an SiC-gebundene Vinylgruppen fördert. Es handelt sich dabei im allgemeinen um Verbindungen oder Komplexe von Platin, wie Platin-Vinylsiloxan-Komplexe, insbesondere Platin-1,3-1,1,3,3-Divinyltetramethyldisiloxan-Komplexe mit oder ohne Gehalt an nachweisbarem anorganischem Halogen, oder um aus $H_2Pt.Cl_6.6H_2O$ und einem Alkanol hergestellte Komplexe.

Vorzugsweise enthalten die erfindungsgemässen Pasten Platin, berechnet als Element und bezogen auf das Gesamtgewicht der jeweiligen Platin enthaltenden und mit den übrigen Bestandteilen der Masse zu vermischenden Paste, in Mengen von 500 Gewichts-ppm (=Gewichtsteil je Million Gewichtsteile) bis 1500 Gewichts-ppm.

Die Diorganopolysiloxane (b) mit mindestens 2 SiC-gebundenen Vinylgruppen je Molekül sind vorzugsweise solche der Formel

$$R_2R'SiO(SiR_2'O)_nSiR'R_2$$

wobei R gleiche oder verschiedene, von aliphatischen Kohlenstoff-Kohlenstoff-Mehrfachbindungen freie, SiC-gebundene organische Reste, R' eine Vinylgruppe bedeutet oder die gleiche Bedeutung wie R hat und n eine ganze Zahl mit einem solchen Wert ist, dass die Viskosität dieser Diorganopolysiloxane bei 25 °C 100 bis 300 000 mPa.s, vorzugsweise 1000 bis 100 000 mPa.s beträgt.

Als organische Reste R sind Kohlenwasserstoffreste mit 1 bis 18 Kohlenstoffatomen je Rest bevorzugt. Beispiele für solche Reste sind Alkylreste, wie der Methyl-, Ethyl-, n-Propyl- und Isopropylrest sowie Octadecylreste; Cycloalkylreste, wie der Cyclohexylrest und Methylcylohexylreste; Arylreste, wie der Phenylrest; Alkarylreste,

wie Tolylreste; und Aralkylreste, wie der Benzylrest und beta-Phenylethylrest. Falls erwünscht, können die Kohlenwasserstoffreste R auch Substituenten enthalten. Diese Substituenten müssen natürlich gegenüber den übrigen Bestandteilen der Zahnabdruckmassen inert sein. Als Beispiele für substituierte Kohlenwasserstoffreste R seien halogenierte Kohlenwasserstoffreste, wie der 3,3,3-Trifluorpropylrest und o-, p- und m-Chlorphenylreste, sowie Cyanalkylreste, wie der beta-Cyanethylrest, genannt. Schon wegen der leichteren Zugänglichkeit sind vorzugsweise mindestens 80% der Anzahl der Reste R Methylreste.

Vorzugsweise haben die von aliphatischen Mehrfachbindungen freien, bei Raumtemperatur flüssigen oder verstreichbaren Kohlenwasserstoffe einen Siedepunkt von mindestens 250°C bei 1000 mbar (abs.). Bevorzugt sind Paraffinöle und Petroleum oder die Verwendung von sowohl Paraffinöl als auch Petroleum.

Die hier in der Beschreibung und mindestens einem der Patentansprüche angegebenen Werte für die Oberfläche von Siliciumdioxyd sind BET-Werte, also Werte, die durch Stickstoffadsorption gemäss ASTM Special Technical Publication No. 51, 1941, Seite 95 ff. bestimmt wurden.

Als Siliciumdioxyd aus dem das hydrophobe Siliciumdioxyd d) hergestellt wird, ist pyrogen erzeugtes Siliciumdioxyd bevorzugt. Es kann aber auch durch Fällung erzeugt sein. Die Hydrophobierung kann durch Umsetzung des Siliciumdioxyds mit Organohalogensilanen, wie Dimethyldichlorsilan, Organoalkyloxysilanen, wie Trimethylethoxysilan, oder Hexaorganodisilazanen, wie Hexamethyldisilazan, erfolgt sein. Insbesondere, wenn ein Hexaorganodisilazan als Hydrophobiermittel verwendet wurde, kann diese Umsetzung in Gegenwart vom Diorganopolysiloxan b) erfolgt sein. Bevorzugt ist mit gasförmiger Organosiliciumverbindung hydrophobiertes Siliciumdioxyd.

Zusätzlich zu den bisher benannten Bestandteilen a) bis d) können die erfindungsgemässen Pasten gegebenenfalls weitere Stoffe enthalten. Beispiele für solche weiteren Stoffe sind Pigmente, lösliche Farbstoffe, Duftstoffe und die Geschwindigkeit der durch Platinkatalysator geförderte Anlagerung von Si-gebundenem Wasserstoff an SiC-gebundene Vinylgruppen regelnde Mittel, wie mindestens 2 SiC-gebundene Vinylgruppen aufweisende Diorgano(poly)siloxane mit einer Viskosität von höchstens 50 $mm^2/s$ bei 25°C, wie 1,3-Divinyl-1,1,3,3-tetramethyldisiloxan.

Mit den übrigen Bestandteilen der Zahnabdruckmassen aus Diorganopolysiloxan mit jeweils eine SiC-gebundene Vinylgruppe aufweisenden Triorganosiloxygruppen als endständigen Einheiten, mindestens 3 Si-gebundene Wasserstoffatome je Molekül enthaltendem Organopolysiloxan, Platin und Füllstoff als wesentlichen Bestandteilen werden die erfindungsgemässen Pasten vorzugsweise in Mengen von 0,5 bis 10 Gewichtsprozent, insbesondere 1 bis 5 Gewichtsprozent, jeweils bezogen auf das Gesamtgewicht der anderen Bestandteile als der Paste, vermischt.

Die anderen Bestandteile als diejenigen der Paste, also die übrigen Bestandteile der Zahnabdruckmassen aus Diorganopolysiloxan mit jeweils eine SiC-gebundene Vinylgruppe aufweisenden Triorganosiloxygruppen als endständigen Einheiten, mindestens 3 Si-gebundene Wasserstoffatome je Molekül enthaltendem Organopolysiloxan, Platin und Füllstoff als wesentlichen Bestandteilen können, natürlich mit Ausnahme des Gehalts an Platin, berechnet als Element, in einer Menge von mindestens 100 Gewichts-ppm, die gleichen sein und in den gleichen Mengen vorliegen wie in den anderen, beispielsweise in US 3 950 300 (ausgegeben 13. April 1956, P. Hittmair et al., Wacker-Chemie GmbH), DE-OS 2 508 560 (ausgegeben 11. September 1975, Dow Corning Ltd.), EP-OS 0 046 907 (veröffentlicht 10. März 1982, Bayer AG), der eingangs genannten US 4 273 902 oder in US 4 096 159 (ausgegeben 20. Juni 1978, W. Hechtl, Wacker-Chemie GmbH) beschriebenen Zahnabdruckmassen. Es erübrigt sich daher, sie hier erneut zu beschreiben. Es sei hier lediglich bemerkt, dass die meisten Vorteile mit dem Gegenstand der Erfindung erzielt werden, wenn die Zahnabdruckmassen vor der Vernetzung teigartige Konsistenz haben, also 1,5 bis 2,5 Teile Füllstoff je Teil des insgesamt enthaltenen Organopolysiloxans enthalten, und dass vor Zusatz der erfindungsgemässen Pasten, welche das Platin enthalten, das mindestens 3 Si-gebundene Wasserstoffatome je Molekül enthaltende Organopolysiloxan und Diorganopolysiloxan mit jeweils eine SiC-gebundene Vinylgruppe aufweisenden Triorganosiloxygruppen als endständigen Einheiten zusammen mit den anderen Bestandteilen als denjenigen der Pasten in einem einzigen Gemisch gelagert werden können, was die Qualitätsprüfung ebenfalls vereinfacht.

Zahnabdruckmassen mit teigartiger Konsistenz aus Diorganopolysiloxan mit jeweils eine SiC-gebundene Vinylgruppe aufweisenden Triorganosiloxygruppen als endständigen Einheiten, mindestens 3 Si-gebundene Wasseratome je Molekül enthaltendem Organopolysiloxan, Platin und Füllstoff als wesentlichen Bestandteilen, die unter Verwendung der erfindungsgemässen Pasten bereitet wurden, eignen sich besonders gut als Dentalvorabdruckmassen.

In den folgenden Beispielen beziehen sich alle Angaben von Teilen und Prozentsätzen bzw. ppm auf das Gewicht, soweit nichts anderes angegeben ist.

Die in den folgenden Beispielen und Vergleichsversuchen verwendete Mischung aus Platin-Vinylsiloxan-Komplex und Verdünnungsmittel wurde hergestellt wie folgt: Zu einer Mischung aus 10 Gewichtsteilen $H_2PtCl_6.6H_2O$, 20 Gewichtsteilen 1,3-Divinyl-1,1,3,3-tetramethyldisiloxan und 50 Gewichtsteilen Ethanol wurden 20 Gewichtsteile Natriumbicarbonat gegeben. Das Gemisch wurde 30 Minuten unter Rühren zum Sieden unter Rückfluss erwärmt, dann 15 Stunden

stehen gelassen und danach filtriert. Aus dem Filtrat wurden bei etwa 16 hPa (abs.) die flüchtigen Bestandteile abdestilliert. Als Rückstand wurden 17 Gewichtsteile einer Flüssigkeit erhalten, die in Benzol gelöst wurde. Die Lösung wurde filtriert und aus dem Filtrat das Benzol abdestilliert. Der Rückstand wurde mit Dimethylvinylsiloxaneinheiten als endständige Einheiten aufweisendem Dimethylpolysiloxan mit einer Viskosität von 1400 mPa.s bei 25°C als Verdünnungsmittel in solcher Menge vermischt, das die Mischung 1 Gewichtsprozent Platin, berechnet als Element, enthält.

Beispiel 1

a) In einem Planetenmischrührwerk werden bei Raumtemperatur und dem Druck der umgebenden Atmosphäre, also etwa 1000 hPa (abs.), 360 Teile wasserfreies, Dimethylvinylsiloxygruppen als endständige Einheiten aufweisendes Dimethylpolysiloxan mit einer Viskosität von 18 000 mPa.s bei 25°C mit 105 Teilen Paraffinöl (pro Analyse), 30 Teilen in Pulverform vorliegendem und mit bei Raumtemperatur flüssigem, durch Trimethylsiloxygruppen endblockiertem Dimethylpolysiloxan angeteigtem, handelsüblichem Weisspigment, 42 Teilen handelsüblichem mit Dimethyldichlorsilan hydrophobiertem Siliciumdioxyd mit einer Oberfläche von 120 ± 30 $m^2/g$ (vgl. Chemiker-Zeitung, 1965, Seite 437 bis 440) und 43 Teilen einer Mischung, die ihrerseits durch Vermischen von

(A) 35 Teilen der 1% Platin, berechnet als Element, enthaltenden Mischung aus Platin-Vinylsiloxan-Komplex und Verdünnungsmittel

(B) 4 Teilen eines durch Trimethylsiloxygruppen endblockierten Diorganopolysiloxans aus 66,66 Molprozent Dimethylsiloxaneinheiten und 33,33 Molprozent Methylvinylsiloxaneinheiten mit einer Viskosität von 10 $mm^2/s$ und

(C) 4 Teilen des Dimethylvinylsiloxygruppen als endständige Einheiten aufweisenden Dimethylpolysiloxans mit einer Viskosität von 18 000 mPa.s bei 25°C bereitet wurde, vermischt. Es wird eine weiche, cremige, weisse, Platin in einer Menge von 600 ppm, berechnet als Element und bezogen auf das Gesamtgewicht der Paste, Organopolysiloxan und Füllstoff enthaltende Paste erhalten. Sie wird vor ihrer endgültigen Verwendung 7 Tage bei Raumtemperatur bzw. 70°C gelagert.

b) 1. In einem Planetenmischrührwerk werden bei Raumtemperatur und 100 hPa (abs.) 834 Teile des wasserfreien Dimethylvinylsiloxygruppen als endständige Einheiten aufweisenden Dimethylpolysiloxans mit einer Viskosität von 18 000 mPa.s bei 25°C und 760 Teile wasserfreier Diatomeenerde vermischt. In die so erhaltene Mischung werden in Anteilen 1370 Teile wasserfreies Cristobalitmehl eingemischt. Die so erhaltene Mischung wird nach Beendigung der Zugabe des Cristobalits noch 1 Stunde bei 100 hPa (abs.) in dem Planetenmischrührwerk und nach Zugabe von 172 Teilen Paraffinöl (pro Analyse) noch 0,5 Stunden bei 100 hPa (abs.) in dem Planetenmischrührwerk bewegt. Danach wird die Mischung 14 Tage bei Raumtemperatur gelagert.

2. 2700 Teile der Mischung, deren Herstellung vorstehend unter 1. beschrieben wurde, werden unter Kneten bei Raumtemperatur und dem Druck der umgebenden Atmosphäre mit 75 Teilen eines wasserfreien, Dimethylhydrogensiloxaneinheiten als endständige Einheiten aufweisenden Diorganopolysiloxans aus Dimethylsiloxaneinheiten und Methylhydrogensiloxaneinheiten, worin 10 Dimethylsiloxaneinheiten je Methylhydrogensiloxaneinheit vorliegen, mit einer Viskosität von 150 mPa.s bei 25°C und 15 Teilen Chromoxid-Grün vermischt. Dann wird eingeschlossene Luft durch Kneten bei 100 hPa (abs.) entfernt. Danach wird die Mischung 7 Tage bei Raumtemperatur bzw. bei 70°C unter Ausschluss von Luft und Wasser gelagert.

3. Ein Teil der Mischung, deren Herstellung vorstehend unter 2. beschrieben wurde, wird mit 2%, bezogen auf sein Gewicht, der gemäss a) hergestellten Paste vermischt, so dass die fertige Masse 12 ppm Pt enthält. Dabei werden zum Vermischen von 32 g der Mischung, deren Herstellung vorstehend beschrieben wurde, mit den 0,64 g der Platin enthaltenden Paste bis zur Erzielung einer einheitlichen Farbe aus dem Grün- und Weisspigment nur 35 Sekunden benötigt. Die so erhaltene Masse wird bei Raumtemperatur vernetzen gelassen.

Beispiel 2

Die in Beispiel 1 unter b) beschriebene Massnahme wird wiederholt mit der Abänderung, dass 1% der gemäss Beispiel 1 a) hergestellten Paste anstelle der 2% dieser Paste verwendet werden, so dass die fertige Masse 6 ppm Pt enthält.

Beispiel 3

Die in Beispiel 1 unter b) 3. beschriebene Massnahme wird wiederholt mit der Abänderung, dass 0,5% der gemäss Beispiel 1 a) hergestellten Paste anstelle der 2% dieser Paste verwendet werden, so dass die fertige Masse 3 ppm Pt enthält.

Vergleichsversuch (a)

a) 290 Teile der Mischung, deren Herstellung in Beispiel 1 unter b) 1. beschrieben wurde, werden mit 2,763 Teilen einer Mischung, die ihrerseits durch Vermischen der Bestandteile (A), (B) und (C) wie in Beispiel 1 angegeben, bereitet wurde. 60 Teilen wasserfreier Diatomeenerde und 15 Teilen in Pulverform vorliegendem und in bei Raumtemperatur flüssigem, durch Trimethylsiloxygruppen endblockiertem Dimethylpolysiloxan angeteigtem, handelsüblichem Weisspigment bei 100 hPa (abs.) verknetet. Die so erhaltene, 75 ppm Pt enthaltende Mischung wird 7 Tage bei Raumtemperatur bzw. 70°C unter Ausschluss von Luft und Wasser gelagert.

b) 1 Teil der Mischung, deren Herstellung vorstehend unter a) beschrieben wurde, wird mit 1 Teil der Mischung, deren Herstellung in Beispiel 1 unter b) 2. beschrieben wurde, vermischt, so dass die fertige Masse 37,5 ppm Platin enthält.

Dabei werden zum Vermischen von 16 g der Mischung, deren Herstellung vorstehend unter a) beschrieben wurde, mit 16 g der anderen Mischung 50 Sekunden benötigt. Die so erhaltene Masse wird bei Raumtemperatur vernetzen gelassen.

Vergleichsversuch (b)

Die in Vergleichsversuch (a) beschriebene Arbeitsweise wird wiederholt mit der Abänderung, dass bei Stufe a) 1,843 Teile der Mischung aus den Bestandteilen (A), (B) und (C) anstelle der 2,763 Teile verwendet werden, so dass die fertige Masse 25 ppm Pt enthält.

Vergleichsversuch (c)

Die in Vergleichsversuch (a) beschriebene Arbeitsweise wird wiederholt mit der Abänderung, dass bei Stufe a) 0,871 Teile der Mischung aus den Bestandteilen (A), (B) und (C) anstelle der 2,763 Teile verwendet werden, so dass die fertige Masse 12,5 ppm Pt enthält.

Die folgende Tabelle beweist, dass durch Verwendung der erfindungsgemässen Pasten die Menge an schwer zugänglichem Platin gesenkt werden kann und eine geringere Änderung des Vernetzungsverhaltens der Masse bei Lagerung der Paste mit den übrigen Bestandteilen der Masse erzielt wird.

Tabelle

| Beispiel bzw. Vergleichsversuch | Pt ppm | Lagerung bei | Topfzeit[1] Sekunden | 6 min[2] | 7 min | 8 min | 9 min | 10 min | 11 min | 12 min | 20 min | 30 min | 60 min | 90 min |
|---|---|---|---|---|---|---|---|---|---|---|---|---|---|---|
| 1 | 12 | RT[3] | 140 | 60 | 68 | 70 | 70 | 70 | | | | | | |
| 1 | 12 | 70 °C | 140 | 60 | 67 | 68 | 68 | 68 | | | | | | |
| 2 | 6 | RT | 135 | 49 | 62 | 65 | 67 | 69 | | | | | | |
| 2 | 6 | 70 °C | 140 | 60 | 68 | 69 | 69 | 69 | | | | | | |
| 3 | 3 | RT | 155 | 43 | 52 | 61 | 68 | 70 | | | | | | |
| 3 | 3 | 70 °C | 150 | 51 | 62 | 69 | 71 | 71 | | | | | | |
| (a) | 37,5 | RT | 165 | 42 | 58 | 68 | 73 | 74 | 74 | 74 | | | | |
| (a) | 37,5 | 70 °C | 160 | 45 | 67 | 63 | 68 | 70 | 71 | 71 | | | | |
| (b) | 25 | RT | 155 | 60 | 71 | 73 | 73 | 73 | | | | | | |
| (b) | 25 | 70 °C | 245 | | | 5 | 13 | 27 | | 39 | 61 | 66 | | |
| (c) | 12,5 | RT | 180 | | 36 | 47 | 55 | 59 | | 65 | 70 | | | |
| (c) | 12,5 | 70 °C | 930 | | | | | | | | | | 37 | 47 |

1) Zeit zwischen Beginn des Vermischens in Stufe b) 3. der Beispiele bzw. in Stufe b) der Vergleichsversuche und merklicher Vernetzung

2) min = jeweils Minuten nach Beginn des Vermischens in Stufe b) 3. der Beispiele bzw. Stufe b) der Vergleichsversuche

3) RT = jeweils Raumtemperatur

**Patentansprüche**

1. Platin, Organopolysiloxan und Füllstoff enthaltende Pasten für die Bereitung von Zahnabdruckmassen aus Diorganopolysiloxan mit jeweils eine SiC-gebundene Vinylgruppe aufweisenden Triorganosiloxygruppen als endständige Einheiten, mindestens 3 Si-gebundene Wasserstoffatome je Molekül enthaltendem Organopolysiloxan, Platin und Füllstoff als wesentlichen Bestandteilen, dadurch gekennzeichnet, dass sie

a) Platin, berechnet als Element und bezogen auf das Gesamtgewicht der jeweiligen Platin enthaltenden und mit den übrigen Bestandteilen der Masse zu vermischenden Paste, in einer Menge von mindestens 100 Gewichts-ppm,

b) 35 bis 75 Gewichtsprozent, bezogen auf das Gesamtgewicht der jeweiligen Platin enthaltenden und mit den übrigen Bestandteilen der Masse zu vermischenden Paste, Diorganopolysiloxan mit mindestens 2 SiC-gebundenen Vinylgruppen je Molekül und einer Viskosität von mindestens 100 mPa.s bei 25 °C,

c) 10 bis 25 Gewichtsprozent, bezogen auf das Gesamtgewicht der jeweiligen Platin enthaltenden und mit den übrigen Bestandteilen der Masse zu vermischenden Paste, von aliphatischen Kohlenstoff-Kohlenstoff-Mehrfachbindungen freie, bei Raumtemperatur flüssige oder verstreichbare Kohlenwasserstoffe und

d) 3 bis 15 Gewichtsprozent, bezogen auf das Gesamtgewicht der jeweiligen Platin enthaltenden und mit den übrigen Bestandteilen der Masse zu vermischenden Paste, hydrophobes Siliciumdioxyd mit einer Oberfläche von mindestens 50 $m^2/g$

enthalten, wobei die Summe der jeweils innerhalb der oben unter a) bis d) angegebenen Bereiche gewählten Prozentsätze einschliesslich der Prozentsätze von gegebenenfalls zusätzlich in der Paste vorliegenden Stoffen 100 Gewichtsprozent beträgt.

2. Pasten nach Anspruch 1, dadurch gekennzeichnet, dass sie Platin, berechnet als Element

und bezogen auf das Gesamtgewicht der jeweiligen Platin enthaltenden und mit den übrigen Bestandteilen der Masse zu vermischenden Paste, in Mengen von 500 Gewichts-ppm bis 1500 Gewichts-ppm enthalten.

## Claims

1. Pastes containing platinum, organopolysiloxane and filler for the preparation of dental impression compositions comprising a diorganopolysiloxane which contains, as terminal units, triorganosiloxy groups each having an SiC-bound vinyl group, an organopolysiloxane containing at least 3 Si-bound hydrogen atoms per molecule, platinum and a filler as essential components, characterized in that they contain

a) platinum, calculated as the element and based on the total weight of the respective paste containing platinum and to be mixed with the other components of the composition, in an amount of at least 100 ppm by weight,

b) 35 to 75 per cent by weight, based on the total weight of the respective paste containing platinum and to be mixed with the other components of the composition, of a diorganopolysiloxane having at least 2 SiC-bound vinyl groups per molecule and having a viscosity of at least 100 mPa.s at 25°C,

c) 10 to 25 per cent by weight, based on the total weight of the respective paste containing platinum and to be mixed with the other components of the composition, of hydrocarbons which are free from aliphatic carbon-carbon multiple bands and are liquid or spreadable at room temperature, and

d) 3 to 15 per cent by weight, based on the total weight of the respective paste containing platinum and to be mixed with the other components of the composition, of hydrophobic silicon dioxide having a surface area of at least 50 $m^2/g$, the sum of the percentages in each case selected within the ranges indicated above under a) to d), including the percentages of the substances optionally additionally present in the paste, being 100 per cent by weight.

2. Pastes according to Claim 1, characterized in that they contain platinum, calculated as the element and based on the total weight of the respective paste containing platinum and to be mixed with the other components of the composition, in amounts of from 500 ppm by weight to 1500 ppm by weight.

## Revendications

1. Pâtes renfermant du platine, un polyorganosiloxane et une charge pour la préparation de compositions pour empreintes dentaires qui contiennent, comme constituants essentiels, un polydiorganosiloxane portant, comme motifs terminaux, des radicaux triorganosilyloxy contenant chacun un radical vinyle directement lié à Si (liaison du type Si-C), un polyorganosiloxane contenant, par molécule, au moins 3 atomes d'hydrogène liés à Si, du platine et une charge, pâtes caractérisées en ce qu'elles contiennent:

a) du platine en une quantité d'au moins 100 ppm en poids, le platine étant considéré à l'état d'élément et la quantité étant rapportée au poids total de la pâte contenant du platine et à mélanger avec les autres constituants de la composition,

b) de 35 à 75% en poids, par rapport au poids total de la pâte contenant du platine et à mélanger avec les autres constituants de la composition, d'un polydiorganosiloxane qui renferme, par molécule, au moins 2 radicaux vinyles directement liés à Si (liaison du type Si-C) et qui a une viscosité d'au moins 100 mPa.s à 25°C,

c) de 10 à 25% en poids, par rapport au poids total de la pâte contenant du platine et à mélanger avec les autres constituants de la composition, d'hydrocarbures sans liaisons multiples carbone-carbone aliphatiques, liquides ou susceptibles d'être étalés à la température ambiante, et

d) de 3 à 15% en poids, par rapport au poids total de la pâte contenant du platine et à mélanger avec les autres constituants de la composition, de silice hydrophobe ayant une surface d'au moins 50 $m^2/g$, la somme des pourcentages choisis dans les intervalles qui viennent d'être indiqués sous a) à d), y compris les pourcentages de substances contenues éventuellement en plus dans la pâte, étant égale à 100% en poids.

2. Pâtes selon la revendication 1 caractérisées en ce qu'elles contiennent une quantité de platine, considéré à l'état d'élément, comprise entre 500 ppm en poids et 1500 ppm en poids par rapport au poids total de la pâte contenant du platine et à mélanger avec les autres constituants de la composition.